# EUROPEAN PATENT SPECIFICATION

(11) **EP 0 239 400 B1**
(45) Date of publication and mention of the grant of the patent: **03.08.1994**
(21) Application number: 87302620.7
(22) Date of filing: 26.03.1987
(51) Int. Cl.: C12N 15/00, C07K 15/06, C12P 21/02

(54) **Recombinant antibodies and methods for their production**
Rekombinante Antikörper und Verfahren zu deren Herstellung
Anticorps recombinants et leurs procédés de production

(30) Priority: 27.03.1986 GB 8607679
(43) Date of publication of application: 30.09.1987
(73) Proprietor: MEDICAL RESEARCH COUNCIL, London W1N 4AL (GB)
(72) Inventor: Winter, Gregory Paul, Cambridge (GB)
(74) Representative: Walton, Seán Malcolm

(56) References cited:
- PROCEEDINGS OF THE NATIONAL ACADEMY OF SCIENCES OF THE USA, vol. 82, April1985, pages 2125-2127; P.P. CHEN et al.: "Possible involvement of human Dminigenes in the first complementarity-determining region of K light chains"
- NATURE, vol. 321, 29th May 1986, pages 522-525; P.T. JONES et al.: "Replacing the complementarity-determining regions in a human antibody with those from a mouse"
- SCIENCE, vol. 239, 25th March 1988, pages 1534-1536; M. VERHOEYEN et al.:
- "Reshaping human antibodies: grafting an antilysozyme activity"

## Description

The present invention relates to altered antibody binding sites, e.g. forming part of an antibody. The present invention also relates to methods for the production of such altered antibody binding sites.

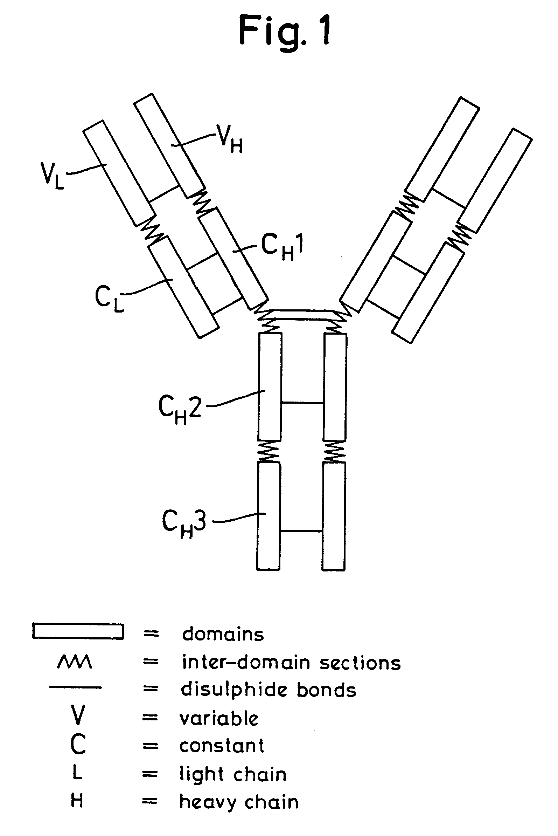

Natural antibodies, or immunoglobulins, comprise two heavy chains linked together by disulphide bonds and two light chains, one light chain being linked to each of the heavy chains by disulphide bonds. The general structure of an antibody of class IgG (i.e. an immunoglobulin (Ig) of class gamma (G)) is shown schematically in Figure 1 of the accompanying drawings.

Each heavy chain has at one end a variable domain followed by a number of constant domains. Each light chain has a variable domain at one end and a constant domain at its other end, the variable domain being aligned with the variable domain of the heavy chain and the constant domain being aligned with the first constant domain of the heavy chain. The constant domains in the light and heavy chains are not involved directly in binding the antibody to the antigen.

The variable domains of each pair of light and heavy chains form the antigen binding site. The domains on the light and heavy chains have the same general structure and each domain comprises four framework regions, whose sequences are relatively conserved, connected by three hypervariable or complementarity determining regions (CDRs) (see Kabat, E.A., Wu, T.T., Bilofsky, H., Reid-Miller, M. and Perry, H., in "Sequences of Proteins of Immunological Interest", US Dept. Health and Human Services 1983). The four framework regions largely adopt a β-sheet conformation and the CDRs form loops connecting, and in some cases forming part of, the β-sheet structure. The CDRs are held in close proximity by the framework regions and, with the CDRs from the other domain, contribute to the formation of the antigen binding site.

For a more detailed account of the structure of variable domains, reference may be made to: Poljak, R.J., Amzel, L.M., Avey, H.P., Chen, B.L., Phizackerly, R.P. and Saul, F., PNAS USA, 70, 3305-3310, 1973; Segal, D.M., Padlan, E.A., Cohen, G.H., Rudikoff, S., Potter, M. and Davies, D.R., PNAS USA, 71, 4298-4302, 1974; and Marquart, M., Deisenhofer, J., Huber, R. and Palm, W., J. Mol. Biol., 141, 369-391, 1980.

In recent years advances in molecular biology based on recombinant DNA techniques have provided processes for the production of a wide range of heterologous polypeptides by transformation of host cells with heterologous DNA sequences which code for the production of the desired products.

EP-A-0 088 994 (Schering Corporation) proposes the construction of recombinant DNA vectors comprising a ds DNA sequence which codes for a variable domain of a light or a heavy chain of an Ig specific for a predetermined ligand. The ds DNA sequence is provided with initiation and termination codons at its 5'- and 3'- termini respectively, but lacks any nucleotides coding for amino acids superfluous to the variable domain. The ds DNA sequence is used to transform bacterial cells. The application does not contemplate variations in the sequence of the variable domain.

EP-A-1 102 634 (Takeda Chemical Industries Limited) describes the cloning and expression in bacterial host organisms of genes coding for the whole or a part of human IgE heavy chain polypeptide, but does not contemplate variations in the sequence of the polypeptide.

EP-A-0 125 023 (Genentech Inc.) proposes the use of recombinant DNA techniques in bacterial cells to produce Ig's which are analogous to those normally found in vertebrate systems and to take advantage of the gene modification techniques proposed therein to construct chimeric Igs or other modified forms of Ig.

The term 'chimeric antibody' is used to describe a protein comprising at least the antigen binding portion of an immunoglobulin molecule (Ig) attached by peptide linkage to at least part of another protein.

It is believed that the proposals set out in the above Genentech application did not lead to the expression of any significant quantities of Ig polypeptide chains, nor to the production of Ig activity, nor to the secretion and assembly of the chains into the desired chimeric Igs.

The production of monoclonal antibodies was first disclosed by Kohler and Milstein (Kohler, G. and Milstein, C., Nature, 256, 495-497, 1975). Such monoclonal antibodies have found widespread use not only as diagnostic reagents (see, for example, 'Immunology for the 80s, Eds. Voller, A., Bartlett, A., and Bidwell, D., MTP Press, Lancaster, 1981) but also in therapy (see, for example, Ritz, J. and Schlossman, S.F., Blood, 59, 1-11, 1982).

The recent emergence of techniques allowing the stable introduction of Ig gene DNA into myeloma cells (see, for example, Oi, V.T., Morrison, S.L., Herzenberg, L.A. and Berg, P., PNAS USA, 80, 825-829, 1983; Neuberger, M.S., EMBO J., 2, 1373-1378, 1983; and Ochi, T., Hawley, R.G., Hawley, T., Schulman, M.J., Traunecker, A., Kohler, G. and Hozumi, N., PNAS USA, 80, 6351-6355, 1983), has opened up the possibility of using in vitro mutagenesis and DNA transfection to construct recombinant Igs possessing novel properties.

However, it is known that the function of an Ig molecule is dependent on its three dimensional structure, which in turn is dependent on its primary amino acid sequence. Thus, changing the amino acid sequence of an Ig may adversely affect its activity. Moreover, a change in the DNA sequence coding for the Ig may affect the ability of the cell containing the DNA sequence to express, secrete or assemble the Ig.

It is therefore not at all clear that it will be possible to produce functional altered antibodies by recombinant DNA techniques.

However, colleagues of the present Inventor have devised a process whereby chimeric antibodies in which both parts of the protein are functional can be secreted. The process, which is disclosed in International Patent Application No. PCT/GB85/00392 (Neuberger et al. and Celltech Limited), comprises:
a) preparing a replicable expression vector including a suitable promoter operably linked to a DNA sequence comprising a first part which encodes at least the variable domain of the heavy or light chain of an Ig molecule and a second part which encodes at least part of a second protein;
b) if necessary, preparing a replicable expression vector including a suitable promoter operably linked to a DNA sequence which encodes at least the variable domain of a complementary light or heavy chain respectively of an Ig molecule;
c) transforming an immortalised mammalian cell line with the or both prepared vectors; and
d) culturing said transformed cell line to produce a chimeric antibody.

The second part of the DNA sequence may encode:
i) at least part, for instance the constant domain of a heavy chain, of an Ig molecule of different species, class or subclass;
ii) at least the active portion or all of an enzyme;
iii) a protein having a known binding specificity;
iv) a protein expressed by a known gene but whose sequence, function or antigenicity is not known; or
v) a protein toxin, such as ricin.

The above Neuberger application only shows the production of chimeric antibodies in which complete variable domains are coded for by the first part of the DNA sequence. It does not show any chimeric antibodies in which the sequence of the variable domain has been altered.

The present invention, in a first aspect, provides an altered antibody binding site comprising complementarity determining regions (hereinafter CDRs) of an Ig light or heavy chain variable domain derived from a first antibody, and framework regions not of said first antibody, wherein antigen binding properties of said first antibody are imparted to the altered antibody binding site by said CDRs.

The determination as to what constitutes a CDR and what constitutes a framework region was made on the basis of the amino-acid sequences of a number of Igs. However, from the three dimensional structure of a number of Igs it is apparent that the antigen binding site of an Ig variable domain comprises three looped regions supported on sheet-like structures. The loop regions do not correspond exactly to the CDRs, although in general there is considerable overlap.

Moreover, not all of the amino-acid residues in the loop regions are solvent accessible and in one case, amino-acid residues in the framework regions are involved in antigen binding (Amit, A.G., Mariuzza, R.A., Phillips, S.E.V. and Poljak, R.J., Science, 233, 747-753, 1986).

It is also known that the variable regions of the two parts of an antigen binding site are held in the correct orientation by inter-chain non-covalent interactions. These may involve amino-acid residues within the CDRs.

Thus, in order to transfer the antigen binding capacity of one variable domain to another, it may not be necessary to replace all of the CDRs with the complete CDRs from the donor variable region. It may be necessary only to transfer those residues which are accessible from the antigen binding site, and this may involve transferring framework region residues as well as CDR residues.

It may also be necessary to ensure that residues essential for inter-chain interactions are preserved in the acceptor variable domain.

Within a domain, the packing together and orientation of the two disulphide bonded β-sheets (and therefore the ends of the CDR loops) are relatively conserved. However, small shifts in packing and orientation of these β-sheets do occur (Lesk, A.M. and Chothia, C., J. Mol. Biol., 160, 325-342, 1982). However, the packing together and orientation of heavy and light chain variable domains is relatively conserved (Chothia, C., Novotny, J., Bruccoleri, R. and Karplus, M., J. Mol. Biol., 186, 651-653, 1985). These points will need to be borne in mind when constructing a new antigen binding site so as to ensure that packing and orientation are not altered to the detriment of antigen binding capacity.

It is thus clear that merely by replacing one or more CDRs with complementary CDRs may not always result in a functional altered antibody. However, given the explanations set out above, it will be well within the competence of the man skilled in the art, either by carrying out routine experimentation or by trial and error testing to obtain a functional altered antibody.

Preferably, the variable domains in both the heavy and light chains have been altered by at least partial CDR replacement and, if necessary, by partial framework region replacement and sequence changing. Although the CDRs may be derived from an antibody of the same class or even subclass as the antibody from which the framework regions are derived, it is envisaged that the CDRs will be derived from an antibody of different class and preferably from an antibody from a different species.

Thus, it is envisaged, for instance, that the CDRs from a mouse antibody could be grafted onto the framework regions of a human antibody. This arrangement will be of particular use in the therapeutic use of monoclonal antibodies.

At present, when a mouse monoclonal antibody or even a chimeric antibody comprising a complete mouse variable domain is injected into a human, the human body's immune system recognises the mouse variable domain as foreign and produces an immune response thereto. Thus, on subsequent injections of the mouse antibody or chimeric antibody into the human, its effectiveness is considerably reduced by the action of the body's immune system against the foreign antibody. In the altered antibody of the present invention, only the CDRs of the antibody will be foreign to the body, and this should minimise side effects if used for human therapy. Although, for example, human and mouse framework regions have characteristic sequences, there seem to be no characteristic features which distinguish human from mouse CDRs. Thus, an antibody comprised of mouse CDRs in a human framework may well be no more foreign to the body than a genuine human antibody.

Even with the altered antibodies of the present invention, there is likely to be an anti-idiotypic response by the recipient of the altered antibody. This response is directed to the antibody binding region of the altered antibody, It is believed that at least some anti-idiotype antibodies are directed at sites bridging the CDRs and the framework regions. It would therefore be possible to provide a panel of antibodies having the same partial or complete CDR replacements but on a series of different framework regions. Thus, once a first altered antibody became therapeutically ineffective, due to an anti-idiotype response, a second altered antibody from the series could be used, and so on, to overcome the effect of the anti-idiotype response. Thus, the useful life of the antigen-binding capacity of the altered antibodies could be extended.

The altered antibody binding site may form part of the structure of a natural antibody or a fragment thereof. Thus, the invention may, for example, provide a complete antibody, an (Fab')₂ fragment, an Fab fragment, a light chain dimer or a heavy chain dimer. Alternatively, the altered antibody binding site may form part of a chimeric antibody of the type described in the Neuberger application referred to above. The production of such an altered chimeric antibody can be carried out using the methods described below used in conjunction with the methods described in the Neuberger application.

The present invention, in a second aspect, comprises a method for producing an altered antibody binding site comprising:
a) preparing a first replicable expression vector including a suitable promoter operably linked to a DNA sequence which encodes at least a variable domain of an Ig heavy or light chain, comprising CDRs derived from a first antibody and framework regions not of said first antibody, whereby antigen binding properties of said first antibody are imparted to the altered antibody binding site by said CDRs;
b) if necessary, preparing a second replicable expression vector including a suitable promoter operably linked to a DNA sequence which encodes at least the varible domain of a complementary Ig light or heavy chain
c) transforming a cell line with the first or both prepared vectors; and
d) culturing said transformed cell line to produce said altered antibody with binding site.

The present invention also includes vectors used to transform the cell line, vectors used in producing the transforming vectors, cell lines transformed with the transforming vectors, cell lines transformed with preparative vectors, and methods for their production.

Preferably, the cell line which is transformed to produce the altered antibody binding site is an immortalised mammalian cell line, which is advantageously of lymphoid origin, such as a myeloma, hybridoma, trioma or quadroma cell line. The cell line may also comprise a normal lymphoid cell, such as a B-cell, which has been immortalised by transformation with a virus, such as the Epstein-Barr virus. Most preferably, the immortalised cell line is a myeloma cell line or a derivative thereof.

Although the cell line used to produce the altered antibody binding site is preferably a mammalian cell line, any other suitable cell line, such as a bacterial cell line or a yeast cell line, may alternatively be used. In particular, it is envisaged that E. coli derived bacterial strains could be used.

It is known that some immortalised lymphoid cell lines, such as myeloma cell lines, in their normal state secrete isolated Ig light or heavy chains. If such a cell line is transformed with the vector prepared in step a) of the process of the invention, it will not be necessary to carry out step b) of the process, provided that the normally secreted chain is complementary to the variable domain of the Ig chain encoded by the vector prepared in step a).

However, where the immortalised cell line does not secrete or does not secrete a complementary chain, it will be necessary to carry out step b). This step may be carried out by further manipulating the vector produced in step a) so that this vector encodes not only the variable domain of an altered antibody light or heavy chain, but also the complementary variable domain.
Alternatively, step b) is carried out by preparing a second vector which is used to transform the immortalised cell line. This alternative leads to easier construct preparation, but may be less preferred than the first alternative in that it may not lead to as efficient production of antibody.

The techniques by which such vectors can be produced and used to transform the immortalised cell lines are well known in the art, and do not form any part of the invention.

In the case where the immortalised cell line secretes a complementary light or heavy chain, the transformed cell line may be produced for example by transforming a suitable bacterial cell with the vector and then fusing the bacterial cell with the immortalised cell line by spheroplast fusion. Alternatively, the DNA may be directly introduced into the immortalised cell line by electroporation.

The DNA sequence encoding the altered variable domain may be prepared by oligonucleotide synthesis. This requires that at least the relevant framework region sequence and CDR sequence(s) are known or can be readily determined. Although determining these sequences, the synthesis of the DNA from oligonucleotides and the preparation of suitable vectors is to some extent laborious, it involves the use of known techniques which can readily be carried out by a person skilled in the art in light of the teaching given here.

If it was desired to repeat this strategy to insert a different antigen binding site, it would only require the synthesis of oligonucleotides encoding the CDR(s), as the framework oligonucleotides can be re-used.

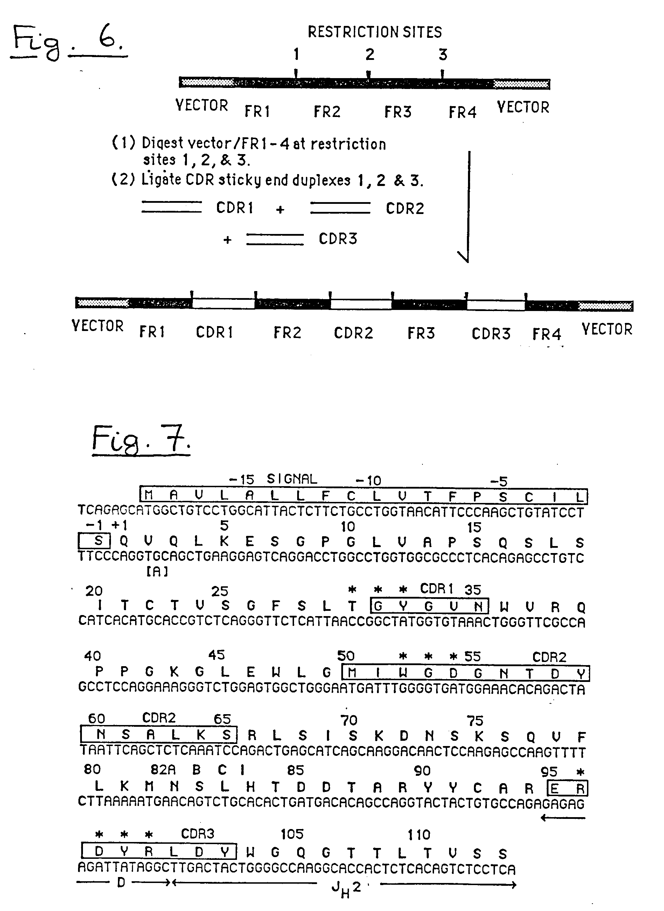

A convenient variant of this technique would involve making a synthetic gene lacking CDR(s) in which the four framework regions are fused together with suitable restriction sites at the junctions. Double stranded synthetic CDR cassettes with sticky ends could then be ligated at the junctions of the framework regions. A protocol for achieving this variant is shown diagrammatically in Figure 6 of the accompanying drawings.

Alternatively, the DNA sequence encoding the altered variable domain may be prepared by primer directed oligonucleotide site-directed mutagenesis. This technique in essence involves hybridising an oligonucleotide coding for a desired mutation with a single strand of DNA containing the region to be mutated and using the single strand as a template for extension of the oligonulcleotide to produce a strand containing the mutation. This technique, in various forms, is described by : Zoller, M.J. and Smith, M., Nuc. Acids Res., 10, 6487-6500, 1982; Norris, K., Norris F., Christiansen, L. and Fiil, N., Nuc. Acids Res., 11, 5103-5112, 1983; Zoller, M.J. and Smith, M., DNA, 3, 479-488 (1984); Kramer, W., Schughart, K. and Fritz, W.-J., Nuc. Acids Res., 10, 6475-6485, 1982.

For various reasons, this technique in its simplest form does not always produce a high frequency of mutation. An improved technique for introducing both single and multiple mutations in an M13 based vector, has been described by Carter et al. (Carter, P., Bedouelle H. and Winter, G., Nuc. Acids Res., 13, 4431-4443, 1985)

Using a long oligonucleotide, it has proved possible to introduce many changes simultaneously (as in Carter et al., loc. cit.) and thus single oligonucleotides, each encoding a CDR, can be used to introduce the three CDRs from a second antibody into the framework regions of a first antibody. Not only is this technique less laborious than total gene synthesis, but it represents a particularly convenient way of expressing a variable domain of required specificity, as it can be simpler than tailoring an entire V_{H} domain for insertion into an expression plasmid.

The oligonucleotides used for site-directed mutagenesis may be prepared by oligonucleotide synthesis or may be isolated from DNA coding for the variable domain of the second antibody by use of suitable restriction enzymes. Such long oligonucleotides will generally be at least 30 bases long and may be up to or over 80 bases in length.

The techniques set out above may also be used, where necessary, to produce the vector of part (b) of the process.

The method of the present invention is envisaged as being of particular use in "humanising" non-human monoclonal antibodies. Thus, for instance, a mouse monoclonal antibody against a particular human cancer cell may be produced by techniques well known in the art. The CDRs from the mouse monoclonal antibody may then be partially or totally grafted into the framework regions of a human monoclonal antibody, which is then produced in quantity by a suitable cell line. The product is thus a specifically targetted, essentially human antibody which will recognise the cancer cells, but will not itself be recognised to any significant degree, by a human's immune system, until the anti-idiotype response eventually becomes apparent. Thus, the method and product of the present invention will be of particular use in the clinical environment.

The present invention is now described, by way of example only, with reference to the accompanying drawings, in which:
Figure 1 is a schematic diagram showing the structure of an IgG molecule;
Figure 2 shows the amino acid sequence of the V_{H} domain of NEWM in comparison with the V_{H} domain of the BI-8 antibody;
Figure 3 shows the amino acid and nucleotide sequence of the HuV_{NP} gene;
Figure 4 shows a comparison of the results for HuV_{NP}-IgE and MoV_{NP}-IgE in binding inhibition assays;
Figure 5 shows the structure of three oligonucleotides used for site directed mutagenesis;
Figure 6 shows a protocol for the construction of CDR replacements by insertion of CDR cassettes into a vector containing four framework regions fused together;
Figure 7 shows the sequence of the variable domain of antibody D1.3 and the gene coding therefor; and
Figure 8 shows a protocol for the cloning of the D1.3 variable domain gene.

### EXAMPLE 1

This example shows the production of an altered antibody in which the variable domain of the heavy chains comprises the framework regions of a human heavy chain and the CDRs from a mouse heavy chain.

The framework regions were derived from the human myeloma heavy chain NEWM, the crystallographic structure of which is known (see Poljak et al., loc. cit. and Reth, M., Hammerling, G.J. and Rajewsky, K., EMBO J., 1, 629-634, 1982.)

The CDRs were derived from the mouse monoclonal antibody B1-8 (see Reth et al., loc. cit.), which binds the hapten NP-cap (4-hydroxy-3-nitrophenyl acetyl-caproic acid: K_{NP-CAP}=1.2 µM).

A gene encoding a variable domain HuV_{NP}, comprising the B1-8 CDRs and the NEWM framework regions, was constructed by gene synthesis as follows.

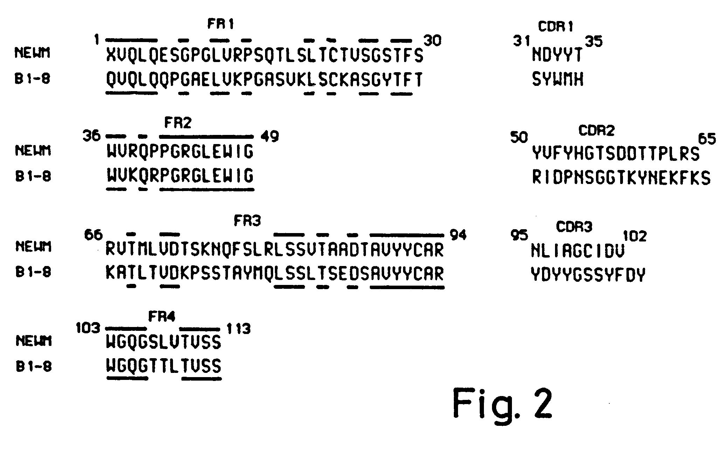

The amino acid sequence of the V_{H} domain of NEWM is shown in Figure 2, wherein it is compared to the amino acid sequence of the V_{H} domain of the B1-8 antibody. The sequence is divided into framework regions and CDRs according to Kabat et al. (loc. cit.). Conserved residues are marked with a line.

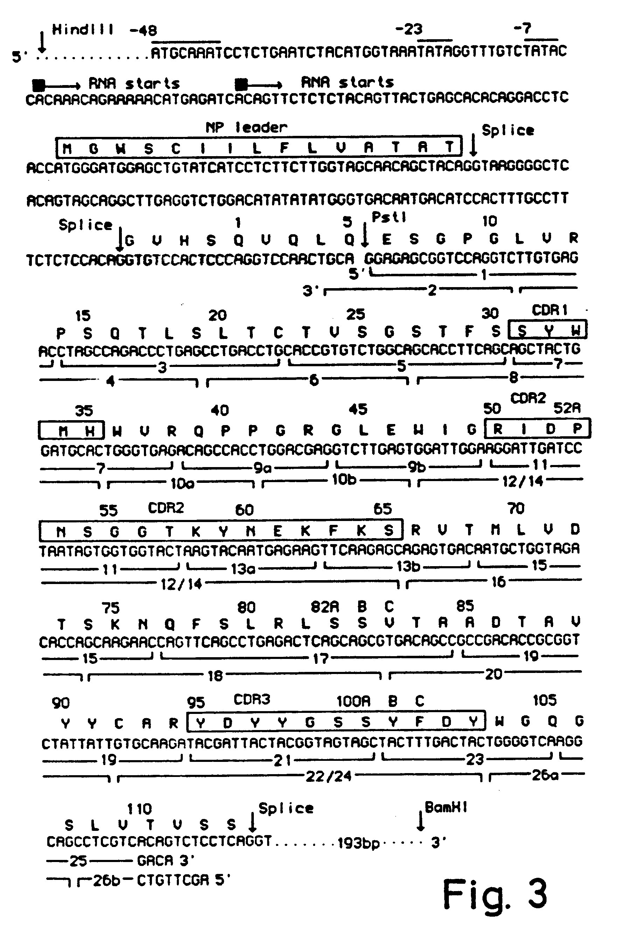

The amino acid and nucleotide sequence of the HuV_{NP} gene, in which the CDRs from the B1-8 antibody alternate with the framework regions of the NEWM antibody, is shown in Figure 3. The HuV_{NP} gene was derived by replacing sections of the MoV_{NP} gene in the vector pSV-V_{NP} (see Neuberger, M.S., Williams, G.T., Mitchell, E.B., Jouhal, S., Flanagan, J.G. and Rabbitts, T.H., Nature, 314, 268-270, 1985) by a synthetic fragment encoding the HuV_{NP} domain. Thus the 5' and 3' non-coding sequences, the leader sequence, the L-V intron, five N-terminal and four C-terminal amino acids are from the MoV_{NP} gene and the rest of the coding sequence is from the synthetic HuV_{NP} fragment.

The oligonucleotides from which the HuV_{NP} fragment was assembled are aligned below the corresponding portion of the HuV_{NP} gene. For convenience in cloning, the ends of oligonucleotides 25 and 26b form a Hind II site followed by a Hind III site, and the sequences of the 25/26b oligonucleotides therefore differ from the HuV_{NP} gene.

The HuV_{NP} synthetic fragment was built as a PstI-Hind III fragment. The nucleotide sequence was derived from the protein sequence using the computer programme ANALYSEQ (Staden, R., Nuc. Acids. Res., 12, 521-538, 1984) with optimal codon usage taken from the sequences of mouse constant domain genes. The oligonucleotides (1 to 26b, 28 in total) vary in size from 14 to 59 residues and were made on a Biosearch SAM or an Applied Biosystems machine, and purified on 8M-urea polyacrylamide gels (see Sanger, F. and Coulson, A., FEBS Lett., 87, 107-110, 1978).

The oligonucleotides were assembled in eight single stranded blocks (A-D) containing oligonucleotides
[1,3,5,7] (Block A), [2,4,6,8] (block A'), [9,11,13a,13b] (Block B), [10a, 10b,12/14] (block B'), [15, 17] (block C), [16,18] (block C'), [19, 21, 23, 25] (block D) and [20, 22/24, 26a, 26b) (block D').

In a typical assembly, for example of block A, 50 pmole of oligonucleotides 1,3,5 and 7 were phosphorylated at the 5' end with T4 polynucleotide kinase and mixed together with 5 pmole of the terminal oligonucleotide [1] which had been phosphorylated with 5 µCi [γ-³²P] ATP (Amersham 3000 Ci/mmole). These oligonucleotides were annealed by heating to 80°C and cooling over 30 minutes to room temperature, with unkinased oligonucleotides 2, 4 and 6 as splints, in 150 µl of 50 mM Tris.Cl, pH 7.5, 10 mM MgCl₂. For the ligation, ATP (1 mM) and DTT (10mM) were added with 50 U T4 DNA ligase (Anglian Biotechnology Ltd.) and incubated for 30 minutes at room temperature. EDTA was added to 10 mM, the sample was extracted with phenol, precipitated from ethanol, dissolved in 20 µl water and boiled for 1 minute with an equal volume of formamide dyes. The sample was loaded onto and run on a 0.3 mm 8M-urea 10% polyacrylamide gel. A band of the expected size was detected by autoradiography and eluted by soaking.

Two full length single strands were assembled from blocks A to D and A' to D' using splint oligonucleotides. Thus blocks A to D were annealed and ligated in 30 µl as set out in the previous paragraph using 100 pmole of oligonucleotides 10a, 16 and 20 as splints. Blocks A' to D' were ligated using oligonucleotides 7, 13b and 17 as splints.

After phenol/ether extraction, block A-D was annealed with block A'-D', small amounts were cloned in the vector M13mp18 (Yanish-Perron, C., Vieira, J. and Messing, J., Gene, 33, 103-119, 1985) cut with PstI and Hind III, and the gene sequenced by the dideoxy technique (Sanger, F., Nicklen, S. and Coulson, A.R., PNAS USA, 74, 5463-5467, 1979).

The MoV_{NP} gene was transferred as a Hind III - BamHI fragment from the vector pSV-V_{NP} (Neuberger et al., loc. cit.) to the vector M13mp8 (Messing, J. and Vieira, J., Gene, 19, 269-276, 1982). To facilitate the replacement of MoV_{NP} coding sequences by the synthetic HuV_{NP} fragment, three Hind II sites were removed from the 5' non-coding sequence by site directed mutagenesis, and a new Hind II site was subsequently introduced near the end of the fourth framework region (FR4 in Figure 2). By cutting the vector with PstI and Hind II, most of the V_{NP} fragment can be inserted as a PstI-Hind II fragment. The sequence at the Hind II site was corrected to NEWM FR4 by site directed mutagenesis.

The Hind III - Bam HI fragment, now carrying the HuV_{NP} gene, was excised from M13 and cloned back into pSV-V_{NP} to replace the MoV_{NP} gene and produce a vector pSV-HuV_{NP}. Finally, the genes for the heavy chain constant domains of human Ig E (Flanagan, J.G. and Rabbitts, T.H., EMBO J., 1, 655-660, 1982) were introduced as a Bam HI fragment to give the vector pSV-HuV_{NP}. HE. This was transfected into the myeloma line J558 L by spheroplast fusion.

The sequence of the HuV_{NP} gene in pSV-HuV_{NP}. HE was checked by recloning the Hind III-Bam HI fragment back into M13mp8 (Messing et al., loc. cit.). J558L myeloma cells secrete lambda l light chains which have been shown to associate with heavy chains containing the MoV_{NP} variable domain to create a binding site for NP-cap or the related hapten NIP-Cap (3-iodo-4-hydroxy-5-nitrophenylacetyl-caproic acid) (Reth, M., Hammerling, G.J. and Rajewsky, K., Eur. J. Immunol., 8, 393-400, 1978).

As the plasmid pSV-HuV_{NP}.HE contains the gpt marker, stably transfected myeloma cells could be selected in a medium containing mycophenolic acid. Transfectants secreted an antibody (HuV_{NP}-IgE) with heavy chains comprising a HuV_{NP} variable domain (i.e. a "humanised" mouse variable region) and human γ constant domains, and lambda l light chains from the J558L myeloma cells.

The culture supernatants of several gpt⁺ clones were assayed by radioimmunoassay and found to contain NIP-cap binding antibody. The antibody secreted by one such clone was purified from culture supernatant by affinity chromatography on NIP-cap Sepharose (Sepharose is a registered trade mark). A polyacrylamide - SDS gel indicated that the protein was indistinguishable from the chimeric antibody MoV_{NP}-IgE (Neuberger et al., loc. cit.).

The HuV_{NP}-IgE antibody competes effectively with the MoV_{NP}-IgE for binding to both anti-human-IgE and to NIP-cap coupled to bovine serum albumin.

Various concentrations of HuV_{NP}-IgE and MoV_{NP}-IgE were used to compete the binding of radiolabelled MoV_{NP}-IgE to polyvinyl microtitre plates coated with (a) Sheep anti-human-IgE antiserum (Seward Laboratories); (b) NIP-cap-bovine serum albumin; (c) Ac38 anti-idiotypic antibody; (d) Ac 146 anti-idiotypic antibody; and (e) rabbit anti-MoV_{NP} antiserum. Binding was also carried out in the presence of MoV_{NP}-IgM antibody (Neuberger, M.S., Williams, G.T. and Fox, R.O., Nature, 312, 604-608, 1984) or of JW5/1/2 which is an IgM antibody differing from the MoV_{NP}-IgM antibody at 13 residues mainly located in the V_{H} CDR2 region.

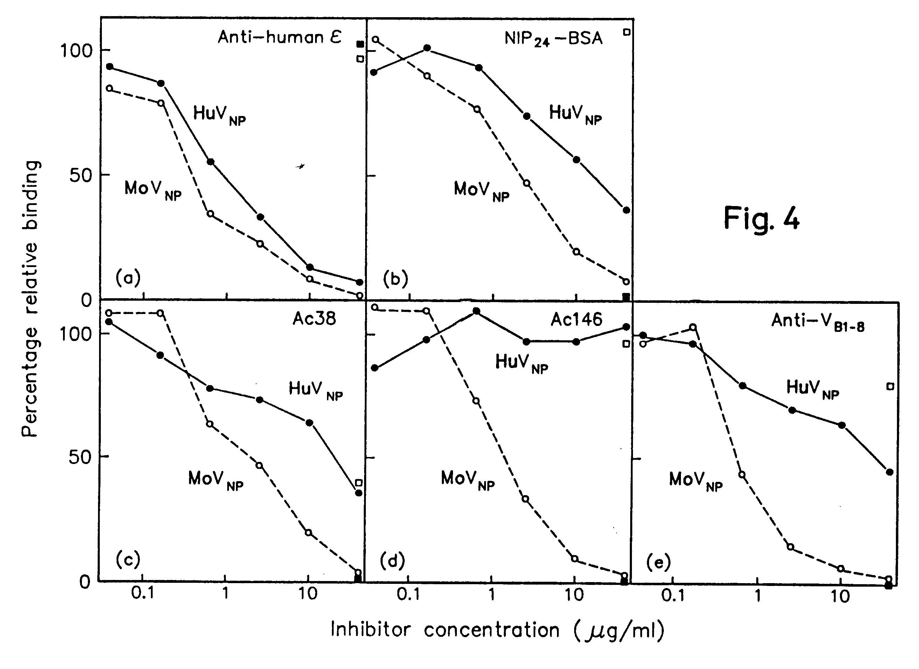

The results of the binding assays are shown in Figure 4, wherein black circles represent HuV_{NP}, white circles MoV_{NP}, black squares MoV_{NP}-IgM and white squares JW5/1/2. Binding is given relative to the binding in the absence of the inhibitor.

The affinities of HuV_{NP}-IgE for NP-cap and NIP-cap were then measured directly using the fluorescence quench technique and compared to those for MoVNP-IgE, using excitation at 295 nm and observing emission at 340 nm (Eisen, H.N., Methods Med. Res., 10, 115-121,1964).

Antibody solutions were diluted to 100 nM in phosphate buffered saline, filtered (0.45 µm pore cellulose acetate) and titrated with NP-cap in the range 0.2 to 20 µM. As a control, mouse DI-3 antibody (Mariuzza, R.A., Jankovic, D.L., Bulot, G., Amit, A.G., Saludjian, P., Le Guern, A., Mazie, J.C. and Poljak, R.J., J. Mol. Biol., 170, 1055-1058, 1983), which does not bind hapten, was titrated in parallel.

Decrease in the ratio of the fluorescence of HuV_{NP}-IgE or MoV_{NP}-IgE to the fluorescence of the D1-3 antibody was taken to be proportional to NP-cap occupancy of the antigen binding sites. The maximum quench was about 40% for both antibodies, and hapten dissociation constants were determined from least-squares fits of triplicate data sets to a hyperbola.

For NIP-cap, hapten concentration varied from 10 to 300 nM, and about 50% quenching of fluorescence was observed at saturation. Since the antibody concentrations were comparable to the value of the dissociation constants, data were fitted by least squares to an equation describing tight binding inhibition (Segal, I.H., in "Enzyme Kinetics", 73-74, Wiley, New York, 1975).

The binding constants obtained from these data for these antibodies are shown in Table 1 below.

**Table 1**

| | K_{NP}-cap | K_{NIP}-cap |
|---|---|---|
| MoV_{NP}-IgE | 1.2 µM | 0.02 µM |
| HuV_{NP}-IgE | 1.9 µM | 0.07 µM |

These results show that the affinities of these antibodies are similar and that the change in affinity is less than would be expected for the loss of a hydrogen bond or a van der Waals contact point at the active site of an enzyme.

Thus, it has been shown that it is possible to produce an antibody specific for an artificial small hapten, comprising a variable domain having human framework regions and mouse CDRs, without any significant loss of antigen binding capacity.

As shown in Figure 4(d), the HuV_{NP}-IgE antibody has lost the MoV_{NP} idiotypic determinant recognised by the antibody Ac146. Furthermore, HuV_{NP}-IgE also binds the Ac38 antibody less well (Figure 4(c)), and it is therefore not surprising that HuV_{NP}-IgE has lost many of the determinants recognised by the polyclonal rabbit anti-idiotypic antiserum (Figure 4(e)).

It can thus be seen that, although the HuV_{NP}-IgE antibody has acquired substantially all the antigen binding capacity of the mouse CDRs, it has not acquired any substantial proportion of the mouse antibody's antigenicity.

The results of Figures 4(d) and 4(e) carry a further practical implication. The mouse (or human) CDRs could be transferred from one set of human frameworks (antibody 1) to another (antibody 2). In therapy, anti-idiotypic antibodies generated in response to antibody 1 might well bind poorly to antibody 2. Thus, as the anti-idiotypic response starts to neutralise antibody 1 treatment could be continued with antibody 2, and the CDRs of a desired specificity used more than once.

For instance, the oligonucleotides encoding the CDRs may be used again, but with a set of oligonucleotides encoding a different set of framework regions.

The above work has shown that antigen binding characteristics can be transferred from one framework to another without loss of activity, so long as the original antibody is specific for a small hapten.

It is known that small haptens generally fit into an antigen binding cleft. However, this may not be true for natural antigens, for instance antigens comprising an epitopic site on a protein or polysaccharide. For such antigens, the antibody may lack a cleft (it may only have a shallow concavity), and surface amino acid residues may play a significant role in antigen binding. It is therefore not readily apparent that the work on artificial antigens shows conclusively that CDR replacement could be used to transfer natural antigen binding properties.

Therefore work was carried out to see if CDR replacement could be used for this purpose. This work also involved using primer-directed, oligonucleotide site-directed mutagenesis using three synthetic oligonucleotides coding for each of the mouse CDRs and the flanking parts of framework regions to produce a variable domain gene similar to the HuV_{NP} gene.

### EXAMPLE 2

The three dimensional structure of a complex of lysozyme and the antilysozyme antibody D1.3 (Amit et al., loc. cit.) was solved by X-ray crystallography. There is a large surface of interaction between the antibody and antigen. The antibody has two heavy chains of the mouse IgGl class (H) and two Kappa light chains (K), and is denoted below as H₂K₂.
The DNA sequence of the heavy chain variable region was determined by making cDNA from the mRNA of the D1.3 hybridoma cells, and cloning into plasmid and M13 vectors. The sequence is shown in Figure 7, in which the boxed residues comprise the three CDRs and the asterisks mark residues which contact lysozyme.

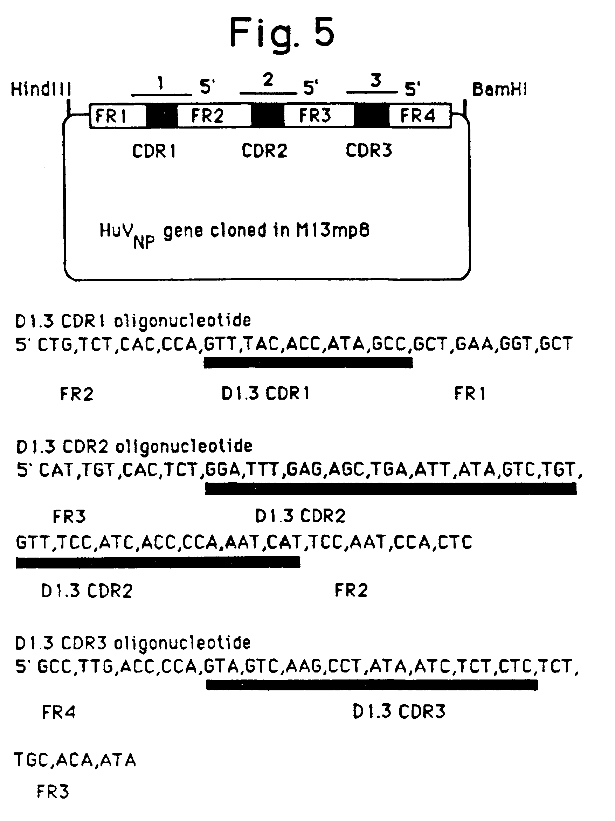

Three synthetic oligonucleotides were then designed to introduce the D1.3 V_{H}CDRs in place of the V_{H}CDRs of the HuV_{NP} gene. The Hu_{NP} gene has been cloned into M13mp8 as a BamHI-Hind III fragment, as described above. Each oligonucleotide has 12 nucleotides at the 5' end and 12 nucleotides at the 3' end which are complementary to the appropriate HuV_{NP} framework regions. The central portion of each oligonucleotide encodes either CDR1, CDR2, or CDR3 of the D1.3 antibody, as shown in Figure 5, to which reference is now made. It can be seen from this Figure that these oligonucleotides are 39, 72 and 48 nucleotides long respectively.

10 pmole of D1.3 CDR1 primer was phosphorylated at the 5' end and annealed to 1µg of the M13-HuV_{NP} template and extended with the Klenow fragment of DNA polymerase in the presence of T4 DNA ligase. After an oligonucleotide extension at 15°C, the sample was used to transfect E. Coli strain BHM71/18 mutL and plaques gridded and grown up as infected colonies.

After transfer to nitrocellulose filters, the colonies were probed at room temperature with 10 pmole of D1.3 CDR1 primer labelled at the 5' end with 30 µCi³²-p-ATP. After a 3˝ wash at 60°C, autoradiography revealed about 20% of the colonies had hybridised well to the probe. All these techniques are fully described in "Oligonucleotide site-directed mutagenesis in M13" an experimental manual by P. Carter, H. Bedouelle, M.M.Y. Waye and G. Winter 1985 and published by Anglian Biotechnology Limited, Hawkins Road, Colchester, Essex CO2 8JX. Several clones were sequenced, and the replacement of HuV_{NP} CDR1 by D1.3 CDR1 was confirmed. This M13 template was used in a second round of mutagenesis with D1.3 CDR2 primer; finally template with both CDRs 1&2 replaced was used in a third round of mutagenesis with D1.3 CDR3 primer. In this case, three rounds of mutagenesis were used.

The variable domain containing the D1.3 CDRs was then attached to sequences encoding the heavy chain constant regions of human IgG2 so as to produce a vector encoding a heavy chain Hu*. The vector was transfected into J558L cells as above. The antibody Hu*₂L₂ is secreted.

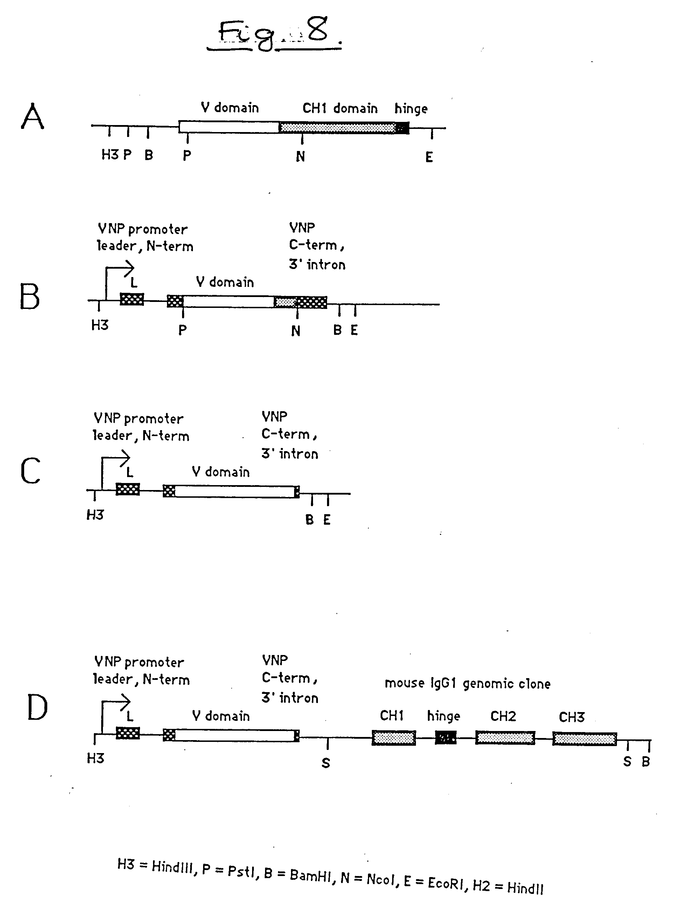

For comparative purposes, the variable region gene for the D1.3 antibody was inserted into a suitable vector and attached to a gene encoding the constant regions of mouse IgGl to produce a gene encoding a heavy chain H* with the same sequence as H. The protocol for achieving this is shown in Figure 8.

As shown in Figure 8, the gene encoding the D1.3 heavy chain V and C_{H}l domains and part of the hinge region are cloned into the M13mp9 vector.

The vector (vector A) is then cut with NcoI, blunted with Klenow polymerase and cut with PstI. The PstI-NcoI fragment is purified and cloned into PstI-HindII cut MV_{NP} vector to replace most of the MV_{NP} coding sequences. The MV_{NP} vector comprises the mouse variable domain gene with its promoter, 5' leader, and 5' and 3' introns cloned into M13mp9. This product is shown as vector B in Figure 8.

Using site directed mutagenesis on the single stranded template of vector B with two primers, the sequence encoding the N-terminal portion of the C_{H}l domain and the PstI site near the N-terminus of the V domain are removed. Thus the V domain of D1.3 now replaces that of V_{NP} to produce vector C of Figure 8.

Vector C is then cut with HindIII and BamHI and the fragment formed thereby is inserted into HindIII/BamHI cut M13mp9. The product is cut with Hind III and SacI and the fragment is inserted into PSV-V_{NP} cut with Hind III/SacI so as to replace the V_{NP} variable domain with the D1.3 variable domain. Mouse IgGl constant domains are cloned into the vector as a SacI fragment to produce vector D of Figure 8.

Vector D of Figure 8 is transfected into J558L cells and the heavy chain H* is secreted in association with the lambda light chain L as an antibody H*₂L₂.

Separated K or L light chains can be produced by treating an appropriate antibody (for instance D1.3 antibody to produce K light chains) with 2-mercaptoethanol in guanidine hydrochloride, blocking the free interchain sulphydryls with iodoacetamide and separating the dissociated heavy and light chains by HPLC in guanidine hydrochloride.

Different heavy and light chains can be reassociated to produce functional antibodies by mixing the separated heavy and light chains, and dialysing into a non-denaturing buffer to promote re-association and refolding. Properly reassociated and folded antibody molecules can be purified on protein A-sepharose columns. Using appropriate combinations of the above procedures, the following antibodies were prepared.
- H₂K₂: (D1 3 antibody)
- H*₂L₂: (D1.3 heavy chain, lambda light chain)
- H*₂K₂: (recombinant equivalent of D1.3)
- Hu*₂L₂: ("humanised" D1.3 heavy chain, lambda light chain)
- Hu*₂K₂: ("humanised" D1.3)
The antibodies containing the lambda light chains were not tested for antigen binding capacity. The other antibodies were, and the results are shown in Table 2.

**Table 2**

| Antibody | Dissociation constant for lysozyme (nM) |
|---|---|
| D1.3 (H₂K₂) | 14.4 |
| D1.3 (H₂K₂) (reassociated) | 15.9, 11.4 |
| recombinant D1.3 (H*₂K₂) (reassociated) | 9.2 |
| "humanised" D1.3 (Hu₂K₂) (reassociated) | 3.5, 3.7 |

The affinity of the antibodies for lysozyme was determined by fluorescent quenching, with excitation at 290nm and emission observed at 340nm Antibody solutions were diluted to 15-30µg/mg in phosphate buffered saline, filtered (0.45 um-cellulose acetate) and titrated with hen eggwhite lysozyme. There is a quenching of fluorescence on adding the lysozyme to the antibody ( >100% quench) and data were fitted by least squares to an equation describing tight binding inhibition (I.H. Segal in Enzyme Kinetics, p73-74, Wiley, New York 1975). Although at first sight the data suggest that the binding of the "humanised" antibody to lysozyme is tighter than in the original D1.3 antibody, this remains to be confirmed. It is clear however that the humanised antibody binds lysozyme with a comparable affinity to D1.3

Further work (with another antibody-CAMPATHl) has shown that CDRs 1,2 and 3 can be exchanged simultaneously, by priming as above with all three primers. 10% hybridisation positives were detected by screening with the CDR1 primer; 30% of these comprised the triple mutant in which all the CDRs were replaced.

It has therefore been shown that CDR replacement can be used not only for artificial antigens (haptens) but also for natural antigens, thereby showing that the present invention will be of therapeutic use.

It will of course be understood that the present invention has been described above purely by way of example, and modifications of detail can be made within the scope of the invention as defined in the appended claims.

## Claims

1. An altered antibody binding site comprising complementarity determining regions (hereinafter CDRs) of an Ig light or heavy chain variable domain derived from a first antibody, and framework regions not of said first antibody, wherein antigen binding properties of said first antibody are imparted to the altered antibody binding site by said CDRs.

2. An altered antibody binding site according to claim 1, wherein CDRs in both the heavy and light chains have been altered.

3. An altered antibody binding site according to claim 1 or 2, comprising CDRs from a mouse antibody and framework regions of a human antibody.

4. An altered antibody binding site according to any one of claims 1 to 3, in which said altered antibody binding site forms part of a natural antibody, a chimaeric antibody or a fragment thereof.

5. A method for producing an altered antibody binding site comprising:
a) preparing a first replicable expression vector including a suitable promoter operably linked to a DNA sequence which encodes at least a variable domain of an Ig heavy or light chain, comprising CDRs derived from a first antibody and framework regions not of said first antibody, whereby antigen binding properties of said first antibody are imparted to the altered antibody binding site by said CDRs;
b) if necessary, preparing a second replicable expression vector including a suitable promoter operably linked to a DNA sequence which encodes at least the variable domain of a complementary Ig light or heavy chain respectively;
c) transforming a cell line with the first or both prepared vectors; and
d) culturing said transformed cell line to produce said altered antibody binding site.

6. A method according to claim 5, further comprising isolating said altered antibody binding site produced in step d).

7. A method according to claim 5 or 6, in which the cell line which is transformed to produce the altered antibody binding site is a mammalian cell line.

8. A method according to claim 7, in which the cell line is a myeloma cell line or a derivative thereof.

9. A method according to any one of claims 5 to 8, in which the DNA sequence encoding the altered variable domain is prepared by use of a synthetic oligonucleotide.

10. The method of any one of claims 5 to 9, in which the DNA sequence encoding the altered variable domain is prepared by primer directed oligonucleotide site-directed mutagenesis using a long oligonucleotide.

## Patentansprüche

1. Modifizierte Antikörperbindungsstelle, umfassend die die Komplementarität bestimmenden Bereiche (nachstehend als CDR bezeichnet) einer variablen Domäne einer leichten oder schweren Ig-Kette, die von einem ersten Antikörper stammen, und Framework-Bereiche, die nicht vom ersten Antikörper stammen, wobei Antigenbindungseigenschaften des ersten Antikörpers der modifizierten Antikörperbindungsstelle durch die CDRs verliehen werden.

2. Modifizierte Antikörperbindungsstelle nach Anspruch 1, wobei die CDRs sowohl in den schweren als auch in den leichten Ketten modifiziert sind.

3. Modifizierte Antikörperbindungsstelle nach Anspruch 1 oder 2, umfassend CDRs eines Maus-Antikörpers und Framework-Bereiche eines menschlichen Antikörpers.

4. Modifizierte Antikörperbindungsstelle nach einem der Ansprüche 1 bis 3, wobei die modifizierte Antikörperbindungsstelle einen Teil eines natürlichen Antikörpers, eines chimären Antikörpers oder eines Fragmentes davon ausmacht.

5. Verfahren zur Herstellung einer modifizierten Antikörperbindungsstelle, umfassend:
a) Herstellung eines ersten replizierbaren Expressionsvektors, umfassend einen geeigneten Promotor, funktionell verbunden mit einer DNA-Sequenz, die mindestens eine variable Domäne einer schweren oder leichten Ig-Kette codiert, umfassend CDRs, die von einem ersten Antikörper stammen, und Framework-Bereiche, die nicht von diesem ersten Antikörper stammen, wobei Antigenbindungseigenschaften des ersten Antikörpers der modifizierten Antikörperbindungsstelle durch die CDRs verliehen werden;
b) falls notwendig, Herstellung eines zweiten replizierbaren Expressionsvektors, umfassend einen geeigneten Promotor funktionell verbunden mit einer DNA-Sequenz, die mindestens die variable Domäne einer komplementären leichten bzw. schweren Ig-Kette codiert;
c) Transformation einer Zellinie mit dem ersten oder mit beiden hergestellten Vektoren; und
d) Züchtung der transformierten Zellinie zur Gewinnung der modifizierten Antikörperbindungsstelle.

6. Verfahren nach Anspruch 5, das außerdem die Isolierung der in Schritt d) hergestellten modifizierten Antikörperbindungsstelle umfaßt.

7. Verfahren nach Anspruch 5 oder 6, wobei die Zellinie, die zur Produktion der modifizierten Antikörperbindungsstelle transformiert wurde, eine Säugerzellinie ist.

8. Verfahren nach Anspruch 7, wobei die Zellinie eine Myelomzellinie oder ein Derivat davon ist.

9. Verfahren nach einem der Ansprüche 5 bis 8, wobei die DNA-Sequenz, die die modifizierte variable Domäne codiert, mit Hilfe eines synthetischen Oligonucleotids hergestellt wird.

10. Verfahren nach einem der Ansprüche 5 bis 9, wobei die DNA-Sequenz, die die modifizierte variable Domäne codiert, durch Primer-gerichtete Oligonucleotid-gerichtete Mutagenese unter Verwendung eines langen Oligonucleotids hergestellt wird.

## Revendications

1. Site de liaison d'anticorps modifié comprenant des régions déterminantes complémentaires (ci-après CDRs) de domaine variable de chaîne légère ou lourde d'une Ig dérivées d'un premier anticorps, et des régions cadre n'appartenant pas audit premier anticorps, dans lequel les propriétés de liaison à l'antigène dudit premier anticorps sont imparties au site de liaison de l'anticorps modifié par lesdites CDRs.

2. Site de liaison d'anticorps modifié selon la revendication 1, dans lequel les CDRs ont été modifiées à la fois dans les chaînes légères et dans les chaînes lourdes.

3. Site de liaison d'anticorps modifié selon la revendication 1 ou 2, comprenant des CDRs provenant d'anticorps de souris et des régions cadre d'anticorps humain.

4. Site de liaison d'anticorps modifié selon l'une quelconque des revendications 1 à 3, dans lequel ledit site de liaison d'anticorps modifié forme une partie d'un anticorps naturel, d'un anticorps chimère, ou d'un fragment.

5. Procédé de production d'un site de liaison d'anticorps modifié comprenant :
a) la préparation d'un premier vecteur d'expression réplicable incluant un promoteur approprié lié de manière opérationnelle à une séquence d'ADN qui code pour au moins un domaine variable d'une chaîne lourde ou légère d'Ig, comprenant des CDRs dérivées d'un premier anticorps et des régions cadre n'appartenant pas audit premier anticorps, dans lequel les propriétés de liaison à l'antigène dudit premier anticorps sont imparties au site de liaison de l'anticorps modifié par lesdites CDRs;
b) si nécessaire, la préparation d'un second vecteur d'expression réplicable incluant une promoteur approprié lié de manière opérationnelle à une séquence d'ADN qui code pour au moins la partie variable d'une chaîne légère ou lourde d'Ig complémentaire, respectivement;
c) la transformation d'une lignée cellulaire par le premier des deux vecteurs préparés; et
d) la culture de ladite lignée cellulaire transformée pour produire ledit site de liaison d'anticorps modifié.

6. Procédé selon la revendication 5, comprenant en outre l'isolement dudit site de liaison d'anticorps modifié produit au cours de l'étape d).

7. Procédé selon la revendication 5 ou 6, dans lequel la lignée cellulaire qui est transformée pour produire le site de liaison d'anticorps modifié est une lignée cellulaire de mammifère.

8. Procédé selon la revendication 7, dans lequel la lignée cellulaire est une lignée cellulaire de myelome ou un de ses dérivés.

9. Procédé selon l'une quelconque des revendications 5 à 8, dans lequel la séquence d'ADN codant pour le domaine variable modifié est préparée au moyen d'un oligonucléotide synthétique.

10. Procédé selon l'une quelconque des revendications 5 à 9, dans lequel la séquence d'ADN codant pour le domaine variable modifié est préparée par mutagénèse dirigée sur un site d'un oligonucléotide, dirigée par une amorce, en utilisant un oligonucléotide à chaîne longue.
